# EUROPEAN PATENT APPLICATION

(11) **EP 2 993 185 A1**
(43) Date of publication of application: **09.03.2016**
(21) Application number: 14183806.0
(22) Date of filing: 05.09.2014
(51) Int. Cl.: C07K 16/28, A61K 39/395

(54) **Monoclonal antibodies for the treatment of osteoporosis, multiple sclerosis and neoplastic diseases**

(71) Applicant: Ruprecht-Karls-Universität, 69117 Heidelberg (DE); Max-Planck-Gesellschaft zur Förderung der Wissenschaften e.V., 80539 München (DE)
(72) Inventor: Offermanns, Stefan, 61231 Bad Nauheim (DE); Swiercz, Jakub, 61231 Bad Nauheim (DE); Worzfeld, Thomas, 61231 Bad Nauheim (DE)
(74) Representative: Cohausz & Florack

(57) **Abstract**

An antibody or functional fragment thereof specifically blocking an epitope located on plexin-B1 for the binding of semaphorin to plexin-B1 that is located in the region of amino acids 20 to 534 of plexin-B1 is useful in the therapy of osteoporosis, multiple sclerosis and neoplastic diseases.

SEQUENCE LISTING

<110> Ruprecht-Karl Universität Heidelberg

<120> Monoclonal antibodies for the treatment of Osteoporosis, Multiple
Sclerosis and neoplastic diseases

<130> 141221EP

<160> 21

<170> BiSSAP 1.2

<210> 1
<211> 2135
<212> PRT
<213> Artificial Sequence

<220>
<223> Plexin-B1

<400> 1
Met Pro Ala Leu Gly Pro Ala Leu Leu Gln Ala Leu Trp Ala Gly Trp
1               5                   10                  15
Val Leu Thr Leu Gln Pro Leu Pro Pro Thr Ala Phe Thr Pro Asn Gly
            20                  25                  30
Thr Tyr Leu Gln His Leu Ala Arg Asp Pro Thr Ser Gly Thr Leu Tyr
        35                  40                  45
Leu Gly Ala Thr Asn Phe Leu Phe Gln Leu Ser Pro Gly Leu Gln Leu
    50                  55                  60
Glu Ala Thr Val Ser Thr Gly Pro Val Leu Asp Ser Arg Asp Cys Leu
65                  70                  75                  80
Pro Pro Val Met Pro Asp Glu Cys Pro Gln Ala Gln Pro Thr Asn Asn
                85                  90                  95
Pro Asn Gln Leu Leu Leu Val Ser Pro Gly Ala Leu Val Val Cys Gly
            100                 105                 110
Ser Val His Gln Gly Val Cys Glu Gln Arg Arg Leu Gly Gln Leu Glu
        115                 120                 125
Gln Leu Leu Leu Arg Pro Glu Arg Pro Gly Asp Thr Gln Tyr Val Ala
    130                 135                 140
Ala Asn Asp Pro Ala Val Ser Thr Val Gly Leu Val Ala Gln Gly Leu
145                 150                 155                 160
Ala Gly Glu Pro Leu Leu Phe Val Gly Arg Gly Tyr Thr Ser Arg Gly
                165                 170                 175
Val Gly Gly Gly Ile Pro Pro Ile Thr Thr Arg Ala Leu Trp Pro Pro
            180                 185                 190
Asp Pro Gln Ala Ala Phe Ser Tyr Glu Glu Thr Ala Lys Leu Ala Val
        195                 200                 205
Gly Arg Leu Ser Glu Tyr Ser His His Phe Val Ser Ala Phe Ala Arg
    210                 215                 220
Gly Ala Ser Ala Tyr Phe Leu Phe Leu Arg Arg Asp Leu Gln Ala Gln
225                 230                 235                 240
Ser Arg Ala Phe Arg Ala Tyr Val Ser Arg Val Cys Leu Arg Asp Gln
                245                 250                 255
His Tyr Tyr Ser Tyr Val Glu Leu Pro Leu Ala Cys Glu Gly Gly Arg
            260                 265                 270
Tyr Gly Leu Ile Gln Ala Ala Ala Val Ala Thr Ser Arg Glu Val Ala
        275                 280                 285
His Gly Glu Val Leu Phe Ala Ala Phe Ser Ser Ala Ala Pro Pro Thr
    290                 295                 300
Val Gly Arg Pro Pro Ser Ala Ala Ala Gly Ala Ser Gly Ala Ser Ala
305                 310                 315                 320
Leu Cys Ala Phe Pro Leu Asp Glu Val Asp Arg Leu Ala Asn Arg Thr
                325                 330                 335
Arg Asp Ala Cys Tyr Thr Arg Glu Gly Arg Ala Glu Asp Gly Thr Glu
            340                 345                 350
Val Ala Tyr Ile Glu Tyr Asp Val Asn Ser Asp Cys Ala Gln Leu Pro
        355                 360                 365
Val Asp Thr Leu Asp Ala Tyr Pro Cys Gly Ser Asp His Thr Pro Ser

370                 375                 380
Pro Met Ala Ser Arg Val Pro Leu Glu Ala Thr Pro Ile Leu Glu Trp
385                 390                 395                 400
Pro Gly Ile Gln Leu Thr Ala Val Ala Val Thr Met Glu Asp Gly His
                405                 410                 415
Thr Ile Ala Phe Leu Gly Asp Ser Gln Gly Gln Leu His Arg Val Tyr
            420                 425                 430
Leu Gly Pro Gly Ser Asp Gly His Pro Tyr Ser Thr Gln Ser Ile Gln
        435                 440                 445
Gln Gly Ser Ala Val Ser Arg Asp Leu Thr Phe Asp Gly Thr Phe Glu
    450                 455                 460
His Leu Tyr Val Met Thr Gln Ser Thr Leu Leu Lys Val Pro Val Ala
465                 470                 475                 480
Ser Cys Ala Gln His Leu Asp Cys Ala Ser Cys Leu Ala His Arg Asp
                485                 490                 495
Pro Tyr Cys Gly Trp Cys Val Leu Leu Gly Arg Cys Ser Arg Arg Ser
            500                 505                 510
Glu Cys Ser Arg Gly Gln Gly Pro Glu Gln Trp Leu Trp Ser Phe Gln
        515                 520                 525
Pro Glu Leu Gly Cys Leu Gln Val Ala Ala Met Ser Pro Ala Asn Ile
    530                 535                 540
Ser Arg Glu Glu Thr Arg Glu Val Phe Leu Ser Val Pro Asp Leu Pro
545                 550                 555                 560
Pro Leu Trp Pro Gly Glu Ser Tyr Ser Cys His Phe Gly Glu His Gln
                565                 570                 575
Ser Pro Ala Leu Leu Thr Gly Ser Gly Val Met Cys Pro Ser Pro Asp
            580                 585                 590
Pro Ser Glu Ala Pro Val Leu Pro Arg Gly Ala Asp Tyr Val Ser Val
        595                 600                 605
Ser Val Glu Leu Arg Phe Gly Ala Val Val Ile Ala Lys Thr Ser Leu
    610                 615                 620
Ser Phe Tyr Asp Cys Val Ala Val Thr Glu Leu Arg Pro Ser Ala Gln
625                 630                 635                 640
Cys Gln Ala Cys Val Ser Ser Arg Trp Gly Cys Asn Trp Cys Val Trp
                645                 650                 655
Gln His Leu Cys Thr His Lys Ala Ser Cys Asp Ala Gly Pro Met Val
            660                 665                 670
Ala Ser His Gln Ser Pro Leu Val Ser Pro Asp Pro Pro Ala Arg Gly
        675                 680                 685
Gly Pro Ser Pro Ser Pro Pro Thr Ala Pro Lys Ala Leu Ala Thr Pro
    690                 695                 700
Ala Pro Asp Thr Leu Pro Val Glu Pro Gly Ala Pro Ser Thr Ala Thr
705                 710                 715                 720
Ala Ser Asp Ile Ser Pro Gly Ala Ser Pro Ser Leu Leu Ser Pro Trp
                725                 730                 735
Gly Pro Trp Ala Gly Ser Gly Ser Ile Ser Ser Pro Gly Ser Thr Gly
            740                 745                 750
Ser Pro Leu His Glu Glu Pro Ser Pro Pro Ser Pro Gln Asn Gly Pro
        755                 760                 765
Gly Thr Ala Val Pro Ala Pro Thr Asp Phe Arg Pro Ser Ala Thr Pro
    770                 775                 780
Glu Asp Leu Leu Ala Ser Pro Leu Ser Pro Ser Glu Val Ala Ala Val
785                 790                 795                 800
Pro Pro Ala Asp Pro Gly Pro Glu Ala Leu His Pro Thr Val Pro Leu
                805                 810                 815
Asp Leu Pro Pro Ala Thr Val Pro Ala Thr Thr Phe Pro Gly Ala Met
            820                 825                 830
Gly Ser Val Lys Pro Ala Leu Asp Trp Leu Thr Arg Glu Gly Gly Glu
        835                 840                 845
Leu Pro Glu Ala Asp Glu Trp Thr Gly Gly Asp Ala Pro Ala Phe Ser
    850                 855                 860
Thr Ser Thr Leu Leu Ser Gly Asp Gly Asp Ser Ala Glu Leu Glu Gly
865                 870                 875                 880
Pro Pro Ala Pro Leu Ile Leu Pro Ser Ser Leu Asp Tyr Gln Tyr Asp
                885                 890                 895
Thr Pro Gly Leu Trp Glu Leu Glu Glu Ala Thr Leu Gly Ala Ser Ser
            900                 905                 910
Cys Pro Cys Val Glu Ser Val Gln Gly Ser Thr Leu Met Pro Val His
        915                 920                 925

Val Glu Arg Glu Ile Arg Leu Leu Gly Arg Asn Leu His Leu Phe Gln
        930                 935                 940
Asp Gly Pro Gly Asp Asn Glu Cys Val Met Glu Leu Glu Gly Leu Glu
945                 950                 955                 960
Val Val Val Glu Ala Arg Val Glu Cys Glu Pro Pro Pro Asp Thr Gln
                965                 970                 975
Cys His Val Thr Cys Gln Gln His Gln Leu Ser Tyr Glu Ala Leu Gln
            980                 985                 990
Pro Glu Leu Arg Val Gly Leu Phe Leu Arg Arg Ala Gly Arg Leu Arg
        995                 1000                1005
Val Asp Ser Ala Glu Gly Leu His Val Val Leu Tyr Asp Cys Ser Val
    1010                1015                1020
Gly His Gly Asp Cys Ser Arg Cys Gln Thr Ala Met Pro Gln Tyr Gly
1025                1030                1035                1040
Cys Val Trp Cys Glu Gly Glu Arg Pro Arg Cys Val Thr Arg Glu Ala
                1045                1050                1055
Cys Gly Glu Ala Glu Ala Val Ala Thr Gln Cys Pro Ala Pro Leu Ile
            1060                1065                1070
His Ser Val Glu Pro Leu Thr Gly Pro Val Asp Gly Gly Thr Arg Val
        1075                1080                1085
Thr Ile Arg Gly Ser Asn Leu Gly Gln His Val Gln Asp Val Leu Gly
    1090                1095                1100
Met Val Thr Val Ala Gly Val Pro Cys Ala Val Asp Ala Gln Glu Tyr
1105                1110                1115                1120
Glu Val Ser Ser Ser Leu Val Cys Ile Thr Gly Ala Ser Gly Glu Glu
                1125                1130                1135
Val Ala Gly Ala Thr Ala Val Glu Val Pro Gly Arg Gly Arg Gly Val
            1140                1145                1150
Ser Glu His Asp Phe Ala Tyr Gln Asp Pro Lys Val His Ser Ile Phe
        1155                1160                1165
Pro Ala Arg Gly Pro Arg Ala Gly Gly Thr Arg Leu Thr Leu Asn Gly
    1170                1175                1180
Ser Lys Leu Leu Thr Gly Arg Leu Glu Asp Ile Arg Val Val Val Gly
1185                1190                1195                1200
Asp Gln Pro Cys His Leu Leu Pro Glu Gln Gln Ser Glu Gln Leu Arg
                1205                1210                1215
Cys Glu Thr Ser Pro Arg Pro Thr Pro Ala Thr Leu Pro Val Ala Val
            1220                1225                1230
Trp Phe Gly Ala Thr Glu Arg Arg Leu Gln Arg Gly Gln Phe Lys Tyr
        1235                1240                1245
Thr Leu Asp Pro Asn Ile Thr Ser Ala Gly Pro Thr Lys Ser Phe Leu
    1250                1255                1260
Ser Gly Gly Arg Glu Ile Cys Val Arg Gly Gln Asn Leu Asp Val Val
1265                1270                1275                1280
Gln Thr Pro Arg Ile Arg Val Thr Val Val Ser Arg Met Leu Gln Pro
                1285                1290                1295
Ser Gln Gly Leu Gly Arg Arg Arg Arg Val Val Pro Glu Thr Ala Cys
            1300                1305                1310
Ser Leu Gly Pro Ser Cys Ser Ser Gln Gln Phe Glu Glu Pro Cys His
        1315                1320                1325
Val Asn Ser Ser Gln Leu Ile Thr Cys Arg Thr Pro Ala Leu Pro Gly
    1330                1335                1340
Leu Pro Glu Asp Pro Trp Val Arg Val Glu Phe Ile Leu Asp Asn Leu
1345                1350                1355                1360
Val Phe Asp Phe Ala Thr Leu Asn Pro Thr Pro Phe Ser Tyr Glu Ala
                1365                1370                1375
Asp Pro Thr Leu Gln Pro Leu Asn Pro Glu Asp Pro Thr Met Pro Phe
            1380                1385                1390
Arg His Lys Pro Gly Ser Val Phe Ser Val Glu Gly Glu Asn Leu Asp
        1395                1400                1405
Leu Ala Met Ser Lys Glu Glu Val Val Ala Met Ile Gly Asp Gly Pro
    1410                1415                1420
Cys Val Val Lys Thr Leu Thr Arg His His Leu Tyr Cys Glu Pro Pro
1425                1430                1435                1440
Val Glu Gln Pro Leu Pro Arg His His Ala Leu Arg Glu Ala Pro Asp
                1445                1450                1455
Ser Leu Pro Glu Phe Thr Val Gln Met Gly Asn Leu Arg Phe Ser Leu
            1460                1465                1470
Gly His Val Gln Tyr Asp Gly Glu Ser Pro Gly Ala Phe Pro Val Ala

                1475                1480                1485
Ala Gln Val Gly Leu Gly Val Gly Thr Ser Leu Leu Ala Leu Gly Val
        1490                1495                1500
Ile Ile Ile Val Leu Met Tyr Arg Arg Lys Ser Lys Gln Ala Leu Arg
1505                1510                1515                1520
Asp Tyr Lys Lys Val Gln Ile Gln Leu Glu Asn Leu Glu Ser Ser Val
                1525                1530                1535
Arg Asp Arg Cys Lys Lys Glu Phe Thr Asp Leu Met Thr Glu Met Thr
            1540                1545                1550
Asp Leu Thr Ser Asp Leu Leu Gly Ser Gly Ile Pro Phe Leu Asp Tyr
        1555                1560                1565
Lys Val Tyr Ala Glu Arg Ile Phe Phe Pro Gly His Arg Glu Ser Pro
    1570                1575                1580
Leu His Arg Asp Leu Gly Val Pro Glu Ser Arg Arg Pro Thr Val Glu
1585                1590                1595                1600
Gln Gly Leu Gly Gln Leu Ser Asn Leu Leu Asn Ser Lys Leu Phe Leu
                1605                1610                1615
Thr Lys Phe Ile His Thr Leu Glu Ser Gln Arg Thr Phe Ser Ala Arg
            1620                1625                1630
Asp Arg Ala Tyr Val Ala Ser Leu Leu Thr Val Ala Leu His Gly Lys
        1635                1640                1645
Leu Glu Tyr Phe Thr Asp Ile Leu Arg Thr Leu Leu Ser Asp Leu Val
    1650                1655                1660
Ala Gln Tyr Val Ala Lys Asn Pro Lys Leu Met Leu Arg Arg Thr Glu
1665                1670                1675                1680
Thr Val Val Glu Lys Leu Leu Thr Asn Trp Met Ser Ile Cys Leu Tyr
                1685                1690                1695
Thr Phe Val Arg Asp Ser Val Gly Glu Pro Leu Tyr Met Leu Phe Arg
            1700                1705                1710
Gly Ile Lys His Gln Val Asp Lys Gly Pro Val Asp Ser Val Thr Gly
        1715                1720                1725
Lys Ala Lys Tyr Thr Leu Asn Asp Asn Arg Leu Leu Arg Glu Asp Val
    1730                1735                1740
Glu Tyr Arg Pro Leu Thr Leu Asn Ala Leu Leu Ala Val Gly Pro Gly
1745                1750                1755                1760
Ala Gly Glu Ala Gln Gly Val Pro Val Lys Val Leu Asp Cys Asp Thr
                1765                1770                1775
Ile Ser Gln Ala Lys Glu Lys Met Leu Asp Gln Leu Tyr Lys Gly Val
            1780                1785                1790
Pro Leu Thr Gln Arg Pro Asp Pro Arg Thr Leu Asp Val Glu Trp Arg
        1795                1800                1805
Ser Gly Val Ala Gly His Leu Ile Leu Ser Asp Glu Asp Val Thr Ser
    1810                1815                1820
Glu Val Gln Gly Leu Trp Arg Arg Leu Asn Thr Leu Gln His Tyr Lys
1825                1830                1835                1840
Val Pro Asp Gly Ala Thr Val Ala Leu Val Pro Cys Leu Thr Lys His
                1845                1850                1855
Val Leu Arg Glu Asn Gln Asp Tyr Val Pro Gly Glu Arg Thr Pro Met
            1860                1865                1870
Leu Glu Asp Val Asp Glu Gly Gly Ile Arg Pro Trp His Leu Val Lys
        1875                1880                1885
Pro Ser Asp Glu Pro Glu Pro Pro Arg Pro Arg Arg Gly Ser Leu Arg
    1890                1895                1900
Gly Gly Glu Arg Glu Arg Ala Lys Ala Ile Pro Glu Ile Tyr Leu Thr
1905                1910                1915                1920
Arg Leu Leu Ser Met Lys Gly Thr Leu Gln Lys Phe Val Asp Asp Leu
                1925                1930                1935
Phe Gln Val Ile Leu Ser Thr Ser Arg Pro Val Pro Leu Ala Val Lys
            1940                1945                1950
Tyr Phe Phe Asp Leu Leu Asp Glu Gln Ala Gln Gln His Gly Ile Ser
        1955                1960                1965
Asp Gln Asp Thr Ile His Ile Trp Lys Thr Asn Ser Leu Pro Leu Arg
    1970                1975                1980
Phe Trp Ile Asn Ile Ile Lys Asn Pro Gln Phe Val Phe Asp Val Gln
1985                1990                1995                2000
Thr Ser Asp Asn Met Asp Ala Val Leu Leu Val Ile Ala Gln Thr Phe
                2005                2010                2015
Met Asp Ala Cys Thr Leu Ala Asp His Lys Leu Gly Arg Asp Ser Pro
            2020                2025                2030

Ile Asn Lys Leu Leu Tyr Ala Arg Asp Ile Pro Arg Tyr Lys Arg Met
        2035                2040                2045
Val Glu Arg Tyr Tyr Ala Asp Ile Arg Gln Thr Val Pro Ala Ser Asp
    2050                2055                2060
Gln Glu Met Asn Ser Val Leu Ala Glu Leu Ser Trp Asn Tyr Ser Gly
2065                2070                2075                2080
Asp Leu Gly Ala Arg Val Ala Leu His Glu Leu Tyr Lys Tyr Ile Asn
                2085                2090                2095
Lys Tyr Tyr Asp Gln Ile Ile Thr Ala Leu Glu Glu Asp Gly Thr Ala
            2100                2105                2110
Gln Lys Met Gln Leu Gly Tyr Arg Leu Gln Gln Ile Ala Ala Ala Val
        2115                2120                2125
Glu Asn Lys Val Thr Asp Leu
    2130                2135

<210> 2
<211> 318
<212> DNA
<213> Artificial Sequence

<220>
<221> source
<222> 1..318
<223> /mol_type="unassigned DNA"
      /note="mAb #19 VL"
      /organism="Artificial Sequence"

<400> 2
gacccagact ccactcactt tgtcggttac cattggacaa ccagcctcta tctcttgcaa      60

gtcaagtcag agcctcttat atagtaatgg aaaaacctat ttgaattggt tattacagag     120

gccaggccag tctccaaagc gcctaatcta tctggtgtct aaactggact ctggagtccc     180

tgacaggttc actggcagtg gatcaggaac agattttaca ctgaaaatca gcagagtgga     240

ggctgaggat ttgggagttt attactgcgt gcaaggtaca cattttcctc acacgttcgg     300

aggggggacc aagctgg                                                    318


<210> 3
<211> 105
<212> PRT
<213> Artificial Sequence

<220>
<223> mAb #19 VL

<400> 3
Thr Gln Thr Pro Leu Thr Leu Ser Val Thr Ile Gly Gln Pro Ala Ser
1               5                   10                  15
Ile Ser Cys Lys Ser Ser Gln Ser Leu Leu Tyr Ser Asn Gly Lys Thr
            20                  25                  30
Tyr Leu Asn Trp Leu Leu Gln Arg Pro Gly Gln Ser Pro Lys Arg Leu
        35                  40                  45
Ile Tyr Leu Val Ser Lys Leu Asp Ser Gly Val Pro Asp Arg Phe Thr
    50                  55                  60
Gly Ser Gly Ser Gly Thr Asp Phe Thr Leu Lys Ile Ser Arg Val Glu
65                  70                  75                  80
Ala Glu Asp Leu Gly Val Tyr Tyr Cys Val Gln Gly Thr His Phe Pro
                85                  90                  95
His Thr Phe Gly Gly Gly Thr Lys Leu
            100                 105

<210> 4
<211> 268
<212> DNA
<213> Artificial Sequence

<220>
<221> source
<222> 1..268
<223> /mol_type="unassigned DNA"
      /note="mAb #19 VH"
      /organism="Artificial Sequence"

<400> 4
tcctgcaagg ctactggcta cacattcagt agctactgga tagagtgggt aaagcagagg      60

cctggacatg gccttgagtg gattggagag attttacctg gaagtggtag tactaactac     120

aatgagaagt tcaagggcaa ggccacattc actgcagata catcctccaa cacagcctac     180

atgcaactca gcagcctgac atctgaggac tctgccgtct attactgtgt aagaaactat     240

ggttactggg gccaagggac tctggtca                                        268


<210> 5
<211> 89
<212> PRT
<213> Artificial Sequence

<220>
<223> mAb #19 VH

<400> 5
Ser Cys Lys Ala Thr Gly Tyr Thr Phe Ser Ser Tyr Trp Ile Glu Trp
1               5                   10                  15
Val Lys Gln Arg Pro Gly His Gly Leu Glu Trp Ile Gly Glu Ile Leu
            20                  25                  30
Pro Gly Ser Gly Ser Thr Asn Tyr Asn Glu Lys Phe Lys Gly Lys Ala
        35                  40                  45
Thr Phe Thr Ala Asp Thr Ser Ser Asn Thr Ala Tyr Met Gln Leu Ser
    50                  55                  60
Ser Leu Thr Ser Glu Asp Ser Ala Val Tyr Tyr Cys Val Arg Asn Tyr
65                  70                  75                  80
Gly Tyr Trp Gly Gln Gly Thr Leu Val
                85

<210> 6
<211> 285
<212> DNA
<213> Artificial Sequence

<220>
<221> source
<222> 1..285
<223> /mol_type="unassigned DNA"
      /note="mAb #527 VL"
      /organism="Artificial Sequence"

<400> 6
gtcagcagga gagaaggtca ctatgagctg caaatccagt cagagtctgc tcaacagtag      60

aacccgaaag aactacttgg cttggtacca gcagaaacca gggcagtctc ctaaactgct     120

gatctactgg gcatccacta gggaatctgg ggtccctgat cgcttcacag gcagtggatc     180

tgggacagat ttcactctca ccatcagcag tgtgcaggct gaagacctgg cagtttatta     240

ctgcaagcaa tcttataatc tgtacacgtt cggagggggg accaa                     285


<210> 7
<211> 95
<212> PRT

<213> Artificial Sequence

<220>
<223> mAb #527 VL

<400> 7
Ser Ala Gly Glu Lys Val Thr Met Ser Cys Lys Ser Ser Gln Ser Leu
1               5                   10                  15
Leu Asn Ser Arg Thr Arg Lys Asn Tyr Leu Ala Trp Tyr Gln Gln Lys
            20                  25                  30
Pro Gly Gln Ser Pro Lys Leu Leu Ile Tyr Trp Ala Ser Thr Arg Glu
        35                  40                  45
Ser Gly Val Pro Asp Arg Phe Thr Gly Ser Gly Ser Gly Thr Asp Phe
    50                  55                  60
Thr Leu Thr Ile Ser Ser Val Gln Ala Glu Asp Leu Ala Val Tyr Tyr
65                  70                  75                  80
Cys Lys Gln Ser Tyr Asn Leu Tyr Thr Phe Gly Gly Gly Thr Lys
                85                  90                  95

<210> 8
<211> 289
<212> DNA
<213> Artificial Sequence

<220>
<221> source
<222> 1..289
<223> /mol_type="unassigned DNA"
      /note="mAb #527 VH"
      /organism="Artificial Sequence"

<400> 8
gatgtcctgc aaggcttctg gctacacctt caccagctac tggataacct gggtgaagca      60

gaggcctgga caaggccttg agtggattgg agatatttat cctggtagtg gtagtactaa     120

ctacaatgag aagttcaaga gcaaggccac actgactgta gacacatcct ccagcacagc     180

ctacatgcag ctcagcagcc tgacatctga ggactctgcg gtctattact gtgcaagagc     240

gggatactac ggtagtagct cctggtttgc ttactggggc caagggacc                289


<210> 9
<211> 96
<212> PRT
<213> Artificial Sequence

<220>
<223> mAb #527 VH

<400> 9
Met Ser Cys Lys Ala Ser Gly Tyr Thr Phe Thr Ser Tyr Trp Ile Thr
1               5                   10                  15
Trp Val Lys Gln Arg Pro Gly Gln Gly Leu Glu Trp Ile Gly Asp Ile
            20                  25                  30
Tyr Pro Gly Ser Gly Ser Thr Asn Tyr Asn Glu Lys Phe Lys Ser Lys
        35                  40                  45
Ala Thr Leu Thr Val Asp Thr Ser Ser Ser Thr Ala Tyr Met Gln Leu
    50                  55                  60
Ser Ser Leu Thr Ser Glu Asp Ser Ala Val Tyr Tyr Cys Ala Arg Ala
65                  70                  75                  80
Gly Tyr Tyr Gly Ser Ser Ser Trp Phe Ala Tyr Trp Gly Gln Gly Thr
                85                  90                  95



<210> 10
<211> 16

<212> PRT
<213> Artificial Sequence

<220>
<223> mAb #19 VL CDR1

<400> 10
Lys Ser Ser Gln Ser Leu Leu Tyr Ser Asn Gly Lys Thr Tyr Leu Asn
1               5                   10                  15

<210> 11
<211> 7
<212> PRT
<213> Artificial Sequence

<220>
<223> mAb #19 VL CDR2

<400> 11
Leu Val Ser Lys Leu Asp Ser
1               5

<210> 12
<211> 9
<212> PRT
<213> Artificial Sequence

<220>
<223> mAb #19 VL CDR3

<400> 12
Val Gln Gly Thr His Phe Pro His Thr
1               5

<210> 13
<211> 5
<212> PRT
<213> Artificial Sequence

<220>
<223> mAb #19 VH CDR1

<400> 13
Ser Tyr Trp Ile Glu
1               5

<210> 14
<211> 17
<212> PRT
<213> Artificial Sequence

<220>
<223> mAb #19 VH CDR2

<400> 14
Glu Ile Leu Pro Gly Ser Gly Ser Thr Asn Tyr Asn Glu Lys Phe Lys
1               5                   10                  15
Gly

<210> 15
<211> 4
<212> PRT
<213> Artificial Sequence

<220>
<223> mAb #19 VH CDR3

<400> 15
Asn Tyr Gly Tyr
1

<210> 16
<211> 17
<212> PRT
<213> Artificial Sequence

<220>
<223> mAb #527 VL CDR1

<400> 16
Lys Ser Ser Gln Ser Leu Leu Asn Ser Arg Thr Arg Lys Asn Tyr Leu
1               5                   10                  15
Ala


<210> 17
<211> 7
<212> PRT
<213> Artificial Sequence

<220>
<223> mAb #527 VL CDR2

<400> 17
Trp Ala Ser Thr Arg Glu Ser
1               5

<210> 18
<211> 8
<212> PRT
<213> Artificial Sequence

<220>
<223> mAb #527 VL CDR3

<400> 18
Lys Gln Ser Tyr Asn Leu Tyr Thr
1               5

<210> 19
<211> 5
<212> PRT
<213> Artificial Sequence

<220>
<223> mAb #527 VH CDR1

<400> 19
Ser Tyr Trp Ile Thr
1               5

<210> 20
<211> 17
<212> PRT
<213> Artificial Sequence

<220>
<223> mAb #527 VH CDR2

<400> 20
Asp Ile Tyr Pro Gly Ser Gly Ser Thr Asn Tyr Asn Glu Lys Phe Lys
1               5                   10                  15
Ser

<210> 21
<211> 12
<212> PRT
<213> Artificial Sequence

<220>
<223> mAb #527 VH CDR3

<400> 21
Ala Gly Tyr Tyr Gly Ser Ser Ser Trp Phe Ala Tyr
1               5                   10

## Description

### FIELD OF THE INVENTION

The invention refers to antibodies or functional fragments thereof specifically binding to plexin-B1 at a specific epitope, their therapeutic use, nucleic acids encoding such antibodies, and vectors comprising these nucleic acids.

### TECHNICAL BACKGROUND OF THE INVENTION

Semaphorins are the largest identified family of axonal guidance proteins and play an important role in the adhesion, migration, proliferation and differentiation of many other cells including tumor cells. They appear as soluble proteins, transmembrane proteins or as proteins bound via a glycosylphosphatidylinositol anchor (GPI-anchor) to cell membranes. The effects of the semaphorins are mediated via binding to plexins.

Plexins comprise a family of transmembrane receptors for semaphorins (1). Based on homologies, plexins are divided into four subfamilies (A to D). The interaction between plexins and semaphorins is mediated via a highly conserved domain, the "sema domain", which includes a seven petaled β-propelled (2-5). The binding of semaphorins to plexins causes the activation of a plurality of signal pathways (6, 7). All plexins bear in their intracellular part a GTPase-activating protein (GTPase-activating protein, GAP) domain that mediates the guanine nucleotide exchange of R-Ras, M-Ras and Rap1. The inhibition of R-Ras was thereby related to the inhibition of signal transduction by integrins. Plexins of the B-subfamily possess at their C-terminus of a PDZ binding-motif interacting stable with the Rho-guanine nucleotide exchange factors (Rho guanine nucleotide exchange factors, RhoGEFs), LARG and PDZ-RhoGEF. An activation of the B-plexins leads to activation of small GTPases RhoA and RhoC.

Osteoporosis has a prevalence of about 30 % in postmenopausal women and is a major risk factor for bone fractures, reduced quality of life and immobilization. At time, the drugs approved in the treatment of osteoporosis include estrogens and selective estrogen-receptor-modulators, bisphosphonates, the anti-RANKL-antibody denosumab, strontium and parathyroid hormone. Despite advances in pharmacotherapy of osteoporosis possibilities for therapy are still insufficient and leave room for new drugs with significant additional benefits.

The ligand for plexin B1, SEMA4D, is highly expressed in osteoclasts (8, 9). Binding of SEMA4D to its receptor, plexin-B1, which is expressed on osteoblasts, leads to activation of the small GTPase RhoA and inhibition of bone formation.

Consistently herewith, mice, in which the gene for SEMA4D or plexin-B1 is switched off, and mice, which specifically express dominant-negative RhoA in osteoblasts, show an increased bone formation (8, 9). An anti-SEMA4D antibody, which blocks the binding of SEMA4D to plexin-B1, prevents bone loss in mouse model for post-menopausal osteoporosis. Therefore, an inhibition of binding of SEMA4D to plexin-B1 by antiplexin-B1 antibodies represents a promising approach for the activation of osteoblasts and increased bone formation, i.e. in order to counteract the bone loss in postmenopausal women. Crucially and unlike most approved anti-osteoporotic drugs, this therapeutic principle is based on the promotion of bone formation and not on inhibition of bone resorption.

Multiple sclerosis (MS) is a neurological disease with the highest prevalence. Among the therapies available at time the disease is often progressive and leads to significant functional limitations. Hence, there is an urgent need for new therapies that are not only aimed at the delay of progression, but are also aimed at the cure of the disease. Currently approved drugs in the treatment of MS are immuno suppressants and immunomodulators such as glucocorticoids, mitoxantrone, azathioprine, cyclophosphamide, β-interferons, glatiramer acetate, i.v. immunoglobulins, dimethyl fumarate, fingolimod, teriflunomide, natalizumab and fampridine. Some of the latest therapies are often related to undesirable effects and require a close patient monitoring.

Plexin-B1 is expressed by microglia, immune effector cells of the central nervous system (10). Binding of SEMA4D to plexin-B1 leads to activation of microglia with increased expression of the iNOS and NO formation.

In course of experimental autoimmune encephalitis (EAE), a mouse model for multiple sclerosis, infiltrating macrophages and T-cells express SEMA4D, which binds to plexin-B1 on microglia and activates them. This process is crucial for the progression of EAE. An anti-SEMA4D antibody blocking the binding of SEMA4D to plexin-B1 lowers the neuroinflammation and progression of EAE (10). Patients suffering from a virus-induced neuroinflammation with demyelination show an infiltration of SEMA4D-positive T cells in the spinal cord and greatly increased concentrations of SEMA4D in the cerebrospinal fluid (10, 11).

Despite significant progress in the treatment of neoplastic diseases existing therapies for many solid tumors continue to display decisive disadvantages. The majority of the new, targeted therapies delay the progression and metastasis only for a relatively short time, so that tumor diseases still represent the second leading cause of death worldwide.

Tumor-associated macrophages (TAMs) play a crucial role in tumor progression. Drugs specifically directed against the communication between TAM and tumor cells are not yet available. TAMs produce SEMA4D promoting both the growth and invasion of tumor cells as well as acting on endothelial cells for the increased tumor angiogenesis (12,13). In accordance herewith tumors, growing in SEMA4D knockout mice, are smaller, metastasize less and are less vascularized than tumors growing in control animals (13).

The problem underlying the invention is to provide a therapeutic principle that allows modification of the interaction between plexins and semaphorins and at the same time providing a therapeutically beneficial effect on several diseases.

### SUMMARY OF THE INVENTION

Surprisingly this problem is solved with an antibody or functional fragment thereof specifically blocking an epitope located on plexin-B1 for the binding of semaphorin to plexin-B1 that is located in the region of amino acids 20 to 534 of plexin-B1. Said antibody or functional fragment thereof specifically comprises
- a light chain variable region being at least 90% identical to SEQ ID Nos: 3 or 7 and a heavy chain variable region being at least 90 % identical to SEQ ID Nos: 5 or 9,
   or
- in the light chain SEQ ID Nos: 10, 11, 12 or SEQ ID Nos: 16, 17 18 and in the heavy chain SEQ ID Nos: 13, 14, 15 or SEQ ID Nos: 19, 20, 21.

Human osteoclasts express endogenous Sema4D, which reduces osteoblast differentiation through Plexin-Bl expressed by osteoblasts. Surprisingly it was found that the antibody of the invention reverses the negative effects of osteoclasts on osteoblast differentiation.

A further subject of the invention is a DNA sequence encoding the antibody or functional fragment thereof of the invention.

According to another aspect, the invention does also refer to an expression vector comprising the aforementioned DNA sequence functionally linked to a promoter, and a cell line comprising the expression vector of the invention.

The invention also refers to the therapeutical application of the antibody of the invention. In particular, it refers to antibody of the invention for use in treating or preventing osteoporosis, multiple sclerosis and neoplastic diseases.

Finally the invention relates to a kit for analytical and/or diagnostic assay comprising the monoclonal antibody or functional fragment thereof of the invention.

Further surprising effects of the antibody of the invention are that it specifically inhibits SEMA4D induced deactivation R-Ras in MCF7 cells and blocks the SEMA4D induced activation of Rho. These effects appear to be dose dependent.

### DESCRIPTION OF THE PREFERRED EMBODIMENTS OF THE INVENTION

Preliminary tests showed that the entire extracellular portion of human plexin-B1 molecule (amino acids 20 to 1491) was not recombinantly producible in a soluble form. Thereupon, variants of the extracellular portion of human plexin-B1 were then examined in regard of recombinant expressibility and solubility. The antibodies of the invention react at a specific epitope in the region of amino acids 20 to 534 of the plexin-B1 protein.

Surprisingly, the high affinity anti-plexin-B1 antibodies of the invention blocking the binding of semaphorins to plexin-B1 are suited as therapeutical means in therapy of osteoporosis, multiple sclerosis and cancer.

Preferably, the antibodies are murine antibodies or humanized antibodies, in particular humanized murine antibodies. According to another preferred embodiment of the invention the antibody is an IgG antibody or a monoclonal antibody.

The antibodies of the invention can be prepared according to standard methods, wherein the plexin-B1 functional fragment used in the example is used as an immunogen. This functional fragment can be obtained, by isolation of the corresponding gene, cloning and recombinant expression in a known manner. Preferably, the antibody according to the invention is obtained by a method described in the example below.

The term "functional fragment" as used herein refers to parts of the monoclonal antibody such as Fab or Fv" fragments, which have the same epitope specificity as the entire antibody of the invention. The different types of functional fragments as well as the preparation of such fragments are known in the art.

The antibody can be used in conventional immunological methods, such as western blot, ELISA, immunofluorescence, radioimmunoassay (RIA).

Antibodies of the invention comprise such antibodies that in the light chain variable region of the antibody are at least 95% or 98% identical to SEQ ID No: 3 or 7; and in the heavy chain variable region of the antibody are at least 95% or 98% identical to SEQ ID No: 5 or 9.

According to a particular embodiment of the invention the antibody or functional fragments thereof comprises one of the light chain variable regions of SEQ ID No: 3 or 7 and one of the heavy chain variable regions of one of SEQ ID No: 5 or 9, preferably antibodies with SEQ ID Nos: 3 and 5 or SEQ ID Nos: 7 and 9.

According to one embodiment of the invention the antibody or functional fragment thereof is an antibody, wherein one amino acid in at least one of the SEQ ID Nos: 10, 11, 12, 13, 14, 15, 16, 17, 18, 19, 20, 21 is substituted, deleted, added or inserted. For example, one amino acid in the three CDRs of the light chain and/or one amino acid in the three CDRs of the heavy chain variable regions is substituted, deleted, added or inserted.

Another subject matter of the invention relates to the anti-plexin-B1 antibody-encoding DNA sequences. The preparation of such DNA sequences can be performed according to routine protocols.

DNA sequences according to the invention can also be inserted into a vector or expression vector. Thus, the present invention also encompasses the DNA sequences comprising vectors or expression vectors. The term "vector" refers to a plasmid (pUC18, pBR322, pBlueScript), a virus or another suitable vehicle. In a preferred embodiment of the invention the DNA sequence according to the invention in an expression vector is functionally linked to regulatory elements that allow their expression in procaryotic or eucaryotic host cells. Such vectors comprise besides the regulatory elements, for example a promoter, typically a replication origin and specific genes allowing the phenotypic selection of a transformed host cell. The regulatory elements for expression in prokaryotes, such as E. coli, include the lac, trp-promoter or T7 promoter, and for the expression in eucaryotes the AOX1 or GAL1 promoter in yeast and the CMV-, SV40, RVS-40 promoter, CMV- or SV40-enhancer for expression in animal cells. Further examples of suitable promoters are the metallothionein 1- and the polyhedron-promoter. Suitable expression vectors for E. coli include, for example pGE-MEX, pUC derivatives and pGEX-2T. Suitable vectors for expression in yeast are pY100 and Ycpad1, for the expression in mammal cells pMSXND, pKCR, pEFBOS, cDM8 and pCEV4. Particularly preferred is the expression vector pLNOH2.

General methods known in the art can be used to construct expression vectors comprising the DNA sequences of the invention and appropriate control sequences. These methods include, for example, in vitro recombination techniques, synthetic techniques and in vivo recombination.

The invention also relates to vectors comprising the above described host cells. These host cells include bacteria (e.g., E. coli strains HB101, DH1, x1776, JM101, JM109, BL21 and SG13009), yeast, preferably S. cerevisiae, insect cells, preferably Sf9 cells, and animal cells, preferably mammalian cells. Preferred mammalian cells are myeloma cells, wherein the mouse myeloma cell line Ag8 K2/c is particularly preferred. Methods of transforming these host cells for phenotypically selection of transformants and for expression of the DNA molecules of the invention using the above described vectors are known in the art.

The antibody of the invention may be administered in a therapeutically effective amount together with a pharmaceutically acceptable carrier, adjuvant or vehicle. An appropriate pharmaceutical composition for the antibody of the invention can be an injectable preparation. Sterile injectable aqueous or oleaginous solutions or suspensions may be formulated according to known proceedings acknowledged in the art.

The antibody of the invention can also be used in universally applicable diagnostics, for example, in western blots and in methods based on immunofluorescence. Hence, the invention also provides kits for analytical and/or diagnostic detection methods including an anti-plexin-B1 antibody according to the invention or functional fragment thereof and, optionally, plexin-B1 or a bond-active part thereof for control purposes. The term "bond-active part" refers to a functional fragment reacting with the antibody, which also reacts with the entire molecule. Depending on the design of the kit, the antibody can be conjugated to another unit, such as a label, and/or can be immobilized on a solid support (substrate). The kit may also comprise a second antibody for the detection of plexin-B1/antibody-complex. The antibody or functional fragment thereof may be free or immobilized on a solid support, such as a plastic cup, test tube, a microtiter plate, a test strip. The kit can also comprise instructions that describe the use of anti-plexin-B1 antibody or functional fragment thereof in a diagnostic assay. The kit, for example, can further comprise suitable reagents for the detection of markers or for marking positive and negative controls, washing solutions, and dilution buffers.

### BRIEF DESCRIPTION OF THE DRAWINGS

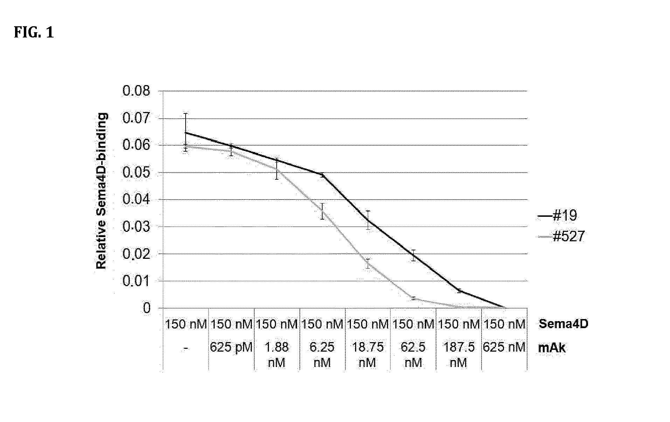

**Fig. 1** shows the effects of murine monoclonal anti-plexin-B1 antibodies on the binding of SEMA4D to plexin-B1-expressing cells. MCF-7 cells expressing endogenous plexin-B1, were incubated for 1 hour with the indicated concentrations of murine monoclonal anti-plexin-B1 antibodies (mAb) No. 19 and No. 527. After washing with PBS, the cells were incubated with myc-SEMA4D for 30 minutes. After removal of unbound myc SEMA4D by washing, the cells were incubated with an HRP-conjugated anti-myc antibody for 30 minutes, washed, and the HRP activity was determined.

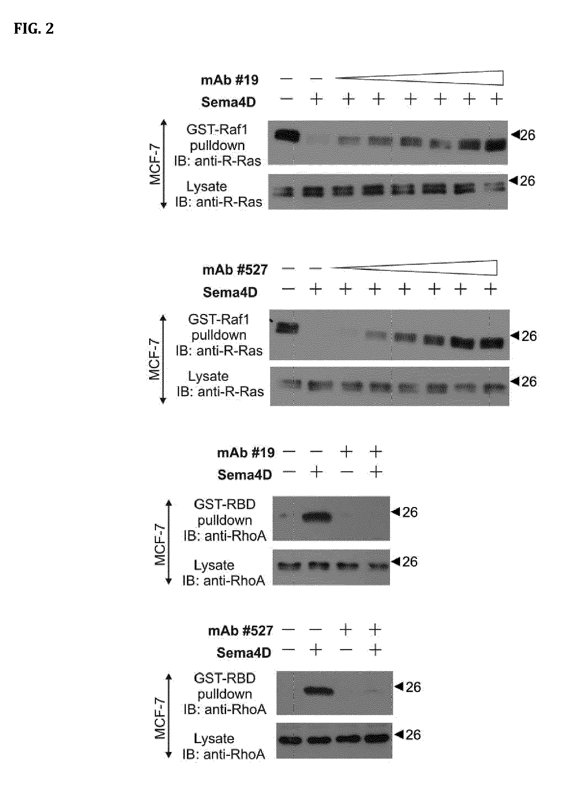

**Fig. 2** shows the effects of murine monoclonal anti-plexin-B1 antibodies on SEMA4D-induced modulation of the activity of RhoA and R-Ras. MCF-7 cells expressing endogenous plexin-B1, were incubated for 1 hour with the monoclonal murine anti-plexin-B1 antibodies (mAb), No. 19 and No. 527. After incubation with SEMA4D for 30 minutes, the cells were lysed and the level of active R-Ras and RhoA was determined (pull-down). The concentration of SEMA4D was 150 nM in all experiments. The concentration of the antibodies in the experiments relating to R-Ras was 625 pM, 1.88 nM, 6.25 nM, 18.75 nM, 62.5 nM, 187.5 nM and 625 nM. The concentration of the antibodies in the experiments relating to RhoA was 150 nM.

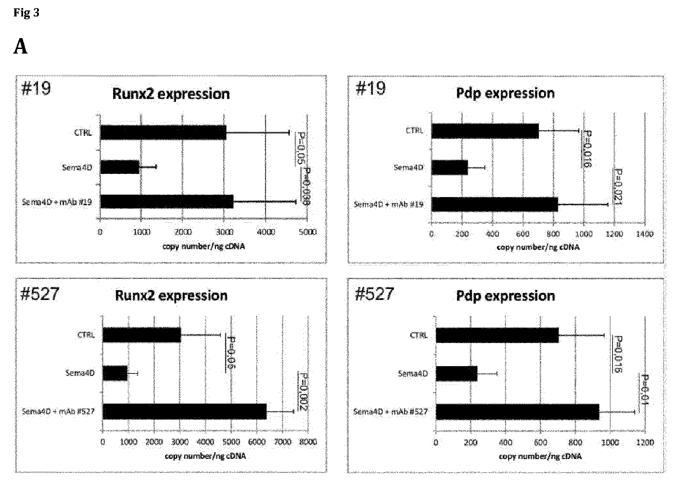

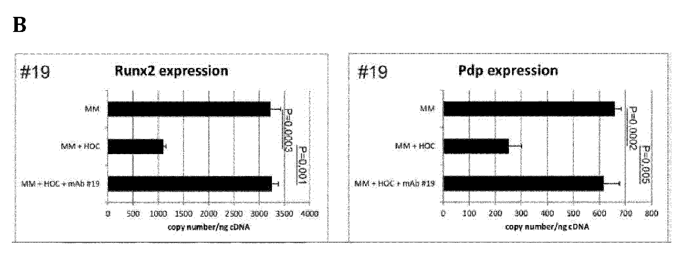

**Fig. 3** shows effects of anti-Plexin-B1 antibodies of the invention on human osteoblast differentiation. (A) Human osteoblasts were cultured in the absence (CTRL) or presence of Sema4D alone or together with the anti-plexin-B1 mAb #19 or #527 (as indicated). After 14 days, expression levels of the osteoblast differentiation markers Runx2 and Pdp were analyzed using qPCR. Stimulation of osteoblasts with Sema4D decreased osteoblast differentiation, and treatment with anti-Plexin-B1 monoclonal antibodies reversed negative effects of Sema4D on osteoblast differentiation. (B) Human osteoblasts were kept in mineralization medium (MM) and cultured in the absence or presence of human osteoclasts (HOC) alone or together with anti-plexin-B1 mAb #19 (as indicated). Expression of Runx2 and Pdp was analyzed as in (A). Human osteoclasts express endogenous Sema4D, which reduces osteoblast differentiation through Plexin-B1 expressed by osteoblasts (Negishi-Koga et al., Nat Med. 2011). Consistently, the inventors observed that a co-culture of human osteoblasts with osteoclasts decreases the expression of differentiation markers in osteoblasts. The present data indicate that the treatment with the monoclonal anti-Plexin-B1 antibody #19 reversed the negative effects of osteoclasts on osteoblast differentiation.

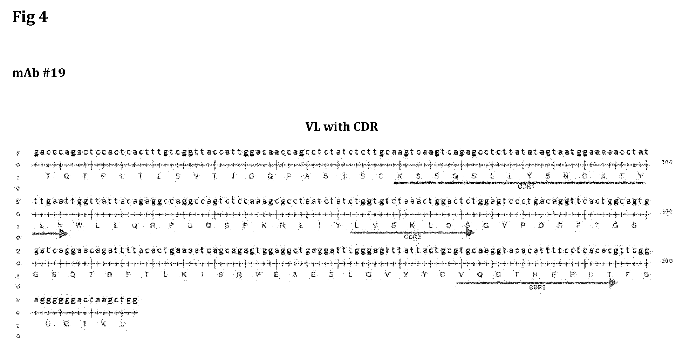

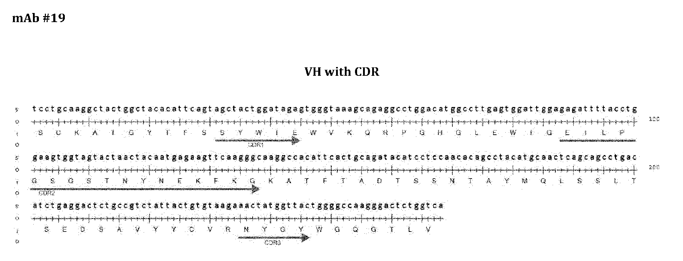

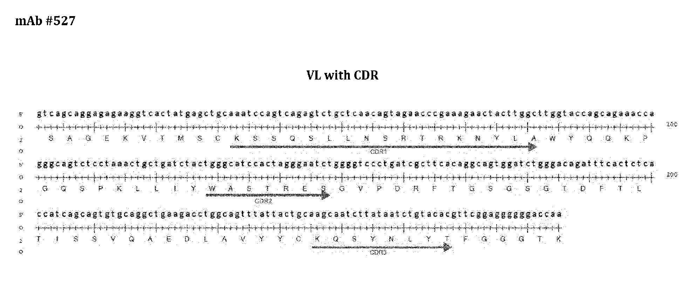

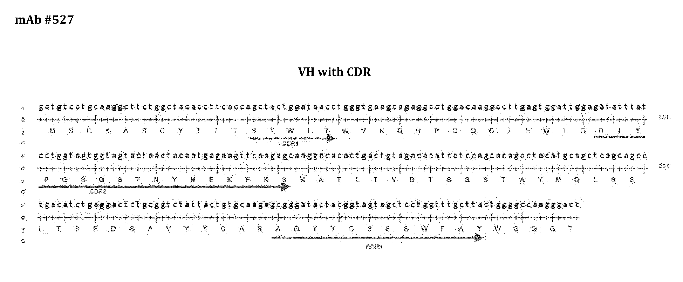

**Fig. 4** shows the amino acid sequences of the variable regions of heavy (VH) and light chain (VL) of the monoclonal antibodies # 19 and # 527. From the monoclonal antibodies # 19 and # 527 producing hybridomas RNA was isolated, transcribed into cDNA, and the variable regions of VL and VH were sequenced. The position of the "complementarity determining regions" CDRs that are underlined in the listings were determined by IgBLAST.

The following example further illustrates the invention. In the examples the antibody mAb #19 comprises in the light chain variable region the CDRs of SEQ ID Nos: 10, 11, 12 and in heavy chain variable region the CDRs of SEQ ID Nos: 13, 14, 15. The antibody mAb #527 comprises in the light chain variable region the CDRs of SEQ ID Nos: 16,17,18 and in the heavy chain variable region the CDRs of SEQ ID Nos: 19, 20, 21.

### EXAMPLE

### Preparation of a plexin-B1-specific antibody according to the invention

In order to immunize mice a secretion construct equipped with a secretion signal based on extensive preliminary studies encoding the extracellular amino acids 20 to 534 of the human plexin-B1 in a soluble form was produced recombinantly. After testing this recombinant protein according to functionality (blockade of the binding of native SEMA4D to plexin-B1, and inhibiting the interaction between plexin-B1 and ErbB-2) mice were immunized and hybridoma clones isolated. Immunization was carried out according to a modified standard immunization protocol (Kohler and Milstein, Nature 256 (1975), 495-497).

| | |
|---|---|
| Days 1 and 5: | Injection of 50 µg of antigen in Freund's complete adjuvant |
| Day 7: | Injection of 50 µg of antigen in Freund's incomplete adjuvant |
| Day 12: | Fusion (with PEG) |

The cell fusions resulted in the generation of specific monoclonal antibodies which were then subcloned.

The testing of 1236 hybridoma supernatants in total by ELISA exhibited positive signals for 163 hybridoma supernatants (binding to the recombinant protein used for immunization). These 163 hybridoma supernatants were further characterized according to their reactivity in a western blot against recombinant plexin-B1, the ability to block the binding of SEMA4D to native plexin-B1, the ability to inhibit the SEMA4D-induced activation of RhoA and the binding of plexin-B1-expressing cells. The anti-plexin-B1 antibodies of the hybridoma supernatants, which tested positive in these assays, were purified using a protein G column and re-assayed.

A detailed characterization of anti-plexin-B1 antibodies revealed that they dose-dependent and specifically block the binding of SEMA4D to MCF-7 cells expressing endogenously Plexin-B1 (Fig. 1). The anti-plexin-B1 antibodies also inhibit the SE-MA4D-induced deactivation of R-Ras in MCF-7 cells in a dose-dependent manner (Fig. 2). In addition, they block the SEMA4D-induced Rho activation (Fig. 2). This effect was also dose-dependent.

The sequences of the variable regions of light and heavy immunoglobulin chains are identified and are available (Fig. 4).

Using various methods (detectability of the resulting 20 to 534 of human plexin-B1 by limited proteolysis of the amino acids fragments in western blot, filter based affinity capturing and elution (FACE) combined with mass spectrometry; peptide mapping) the epitopes recognized by these antibodies were determined.

### NON PATENT LITERATURE CITED IN THE APPLICATION

1. Yazdani, U., and Terman, J.R. 2006. The semaphorins. Genome Biol 7:211.
2. Hota, P.K., and Buck, M. 2012. Plexin structures are coming: opportunities for multilevel investigations of semaphorin guidance receptors, their cell signaling mechanisms, and functions. Cell Mol Life Sci 69:3765-3805.
3. Janssen, B.J., Robinson, R.A., Perez-Branguli, F., Bell, C.H., Mitchell, K.J., Siebold, C., and Jones, E.Y. 2010. Structural basis of semaphorin-plexin signalling. Nature 467:1118-1122.
4. Liu, H., Juo, Z.S., Shim, A.H., Focia, P.J., Chen, X., Garcia, K.C., and He, X. 2010. Structural basis of semaphorin-plexin recognition and viral mimicry from Sema7A and A39R complexes with PlexinC1. Cell 142:749-761.
5. Nogi, T., Yasui, N., Mihara, E., Matsunaga, Y., Noda, M., Yamashita, N., Toyofuku, T., Uchiyama, S., Goshima, Y., Kumanogoh, A., et al. 2010. Structural basis for semaphorin signalling through the plexin receptor. Nature 467:1123-1127.
6. Franco, M., and Tamagnone, L. 2008. Tyrosine phosphorylation in semaphorin signalling: shifting into overdrive. EMBO Rep 9:865-871.
7. Puschel, A.W. 2007. GTPases in semaphorin signaling. Adv Exp Med Biol 600:12-23.
8. Dacquin, R., Domenget, C., Kumanogoh, A., Kikutani, H., Jurdic, P., and Machuca-Gayet, I. 2011. Control of bone resorption by semaphorin 4D is dependent on ovarian function. PLoS One 6:e26627.
9. Negishi-Koga, T., Shinohara, M., Komatsu, N., Bito, H., Kodama, T., Friedel, R.H., and Takayanagi, H. 2011. Suppression of bone formation by osteoclastic expression of semaphorin 4D. Nat Med 17:1473-1480.
10. Okuno, T., Nakatsuji, Y., Moriya, M., Takamatsu, H., Nojima, S., Takegahara, N., Toyofuku, T., Nakagawa, Y., Kang, S., Friedel, R.H., et al. 2010. Roles of SEMA4D-plexin-B1 interactions in the central nervous system for pathogenesis of experimental autoimmune encephalomyelitis. Immunol 184:1499-1506.
11. Giraudon, P., Vincent, P., Vuaillat, C., Verlaeten, O., Cartier, L., Marie-Cardine, A., Mutin, M., Bensussan, A., Belin, M.F., and Boumsell, L. 2004. Semaphorin CD100 from activated T lymphocytes induces process extension collapse in oligodendrocytes and death of immature neural cells. J Immunol 172:1246-1255.
12. Basile, J.R., Castilho, R.M., Williams, V.P., and Gutkind, J.S. 2006. Semaphorin 4D provides a link between axon guidance processes and tumor-induced angiogenesis. Proc Natl Acad Sci U S A 103:9017-9022.
13. Sierra, J.R., Corso, S., Caione, L., Cepero, V., Conrotto, P., Cignetti, A., Piacibello, W., Kumanogoh, A., Kikutani, H., Comoglio, P.M., et al. 2008. Tumor angiogenesis and progression are enhanced by SEMA4D produced by tumor-associated macrophages. J Exp Med 205:1673-1685.

## Claims

1. Antibody or functional fragment thereof specifically blocking an epitope located on plexin-B1 for the binding of semaphorin to plexin-B1 that is located in the region of amino acids 20 to 534 of plexin-B1 and
- having a light chain variable region being at least 90% identical to SEQ ID Nos: 3 or 7 and a heavy chain variable region being at least 90 % identical to SEQ ID Nos: 5 or 9
or
- having in the light chain SEQ ID Nos: 10, 11, 12 or SEQ ID Nos: 16,17 18 and in the heavy chain SEQ ID Nos: 13, 14, 15 or SEQ ID Nos: 19, 20, 21.

2. Antibody or functional fragment thereof according to claim 1, wherein one amino acid in at least one of the SEQ ID Nos: 10, 11, 12, 13, 14, 15, 16, 17, 18, 19, 20, 21 is substituted, deleted, added or inserted.

3. Antibody or functional fragment thereof according to claim 1, wherein the light chain variable region is selected from SEQ ID No: 3 and the heavy chain variable region is selected from SEQ ID No: 5, or the light chain variable region is selected from SEQ ID No: 7 and the heavy chain variable region is selected from SEQ ID No: 9.

4. Antibody or functional fragment thereof according to claim 1, wherein the CDRs of the light chain variable region are those of SEQ ID Nos: 10, 11, 12 and the CDRs of the heavy chain variable region are those of SEQ ID Nos: 13, 14, 15, or wherein the CDRs of the light chain variable region are those of SEQ ID Nos: 16, 17, 18 and the CDRs of the heavy chain variable region are those of SEQ ID Nos: 19, 20, 21.

5. Antibody or functional fragment thereof according to anyone of claims 1 to 4, **characterized in that** the antibody is a monoclonal antibody.

6. Antibody according to anyone of claims 1 to 4, wherein the antibody is a murine antibody or a humanized antibody.

7. Antibody according to anyone of claims 1 to 4, wherein the antibody is an IgG antibody.

8. Antibody or functional fragment thereof according to anyone of claims 1 to 4,
wherein the antibody is **characterized by** specifically inhibiting SEMA4D induced deactivation R-Ras in MCF7 cells and blocking of SEMA4D induced activation of Rho.

9. DNA sequence encoding the antibody or functional fragment thereof of any one of claims 1 to 4.

10. Expression vector comprising the DNA sequence of claim 9 functionally linked to a promoter.

11. Cell line comprising the expression vector according to claim 10.

12. The cell line according to claim 11, wherein the cell line is a murine myeloma cell line.

13. Kit for analytical and/or diagnostic assay comprising a monoclonal antibody or functional fragment thereof according to any one of claims 1 to 8.

14. The antibody or functional fragment thereof of claims 1 to 8 for use in therapy.

15. The antibody or functional fragment thereof of claims 1 to 8 for use in treating or preventing osteoporosis, multiple sclerosis and neoplastic diseases.
